# EUROPEAN PATENT SPECIFICATION

(11) **EP 4 595 904 B1**
(45) Date of publication and mention of the grant of the patent: **25.11.2026**
(21) Application number: 25155372.3
(22) Date of filing: 31.01.2025
(51) Int. Cl.: A61B 17/064, A61B 17/10, A61B 17/068

(54) **BONE STAPLE DELIVERY DEVICES**
VORRICHTUNGEN ZUM SETZEN VON KNOCHENKLAMMERN
DISPOSITIFS DE POSE D'AGRAFES OSSEUSES

(30) Priority: 31.01.2024 US 202418428491
(43) Date of publication of application: 06.08.2025
(73) Proprietor: Primo Medical Group, Stoughton, Massachusetts 02072 (US)
(72) Inventor: COBB, John, Stoughton, 02072 (US); RODGERS, Richard, Hudson, 01749 (US)
(74) Representative: Dummett Copp LLP

(56) References cited:
- WO-A1-2019/231622
- US-A1- 2010 133 316
- US-A1- 2015 230 843
- US-A1- 2019 192 160

## Description

### FIELD

The present disclosure relates to medical devices, particularly bone staple delivery devices, bone staples and methods of use thereof.

### BACKGROUND

Bone staples are used to hold a fractured bone together, particularly by providing a compression force which urges the adjacent bone segments on each side of the fracture towards one another. The staple generally comprises a crown, with two legs at opposing ends of the crown. The staple is disposed in the bone, with the crown spanning the fracture and each of the legs residing in one of the adjacent bone segments.

Prior to being disposed in the bone segments, the staple is placed into a delivery device, which splays the legs away from one another with elastic deformation of the staple. When the staple is released from the delivery device, the elastic memory force of the staple causes the legs to elastically recover towards one another. The force associated with the staple legs being urged back towards one another due to elastic memory provides the compression force which urges the adjacent bone segments on each side of the fracture towards one another.

US 2010/133316 A1 discloses an instrument for fitting compression staples.

### SUMMARY

The present disclosure provides a medical device, comprising a bone staple delivery device. The bone staple delivery device comprises an elongated platform and a lever-actuator. The platform and the lever-actuator form a staple clamp to clamp a bone staple. The lever-actuator has a lever-handle and a lever-head, with the lever-handle rotatable relative to the platform and having a distal end face comprising a planar distal end face and an arcuate distal end face. The lever-head has a planar proximal end face.

When the staple is installed in the clamp staple and the lever-handle is rotated with a closure force in a first direction towards to the platform, the bone staple delivery device is operable such that the arcuate distal end face of the lever-handle contacts the planar proximal end face of the lever-head and moves the lever-head distally relative to the platform to close the staple clamp on the staple.

When the lever-handle is further rotated with the closure force in the first direction towards to the platform, the planar distal end face of the lever-handle and the planar proximal end face of the lever-head come into parallel alignment facing one another, which inhibits the lever-handle from rotating in a second direction away from the platform upon removal of the closure force.

### BRIEF DESCRIPTION OF THE DRAWINGS

The above-mentioned and other features of this disclosure, and the manner of attaining them, will become more apparent and better understood by reference to the following description of embodiments described herein taken in conjunction with the accompanying drawings, wherein:
FIG. 1 is a perspective view of a medical device according to the present disclosure, comprising a bone staple delivery device and a staple;
FIG. 2A is an enlarged side view of the staple of FIG. 1;
FIG. 2B is an enlarged side view of another staple according to the present disclosure;
FIG. 3 is close-up perspective view of a distal region of the bone staple delivery device of FIG. 1;
FIG. 4 is another close-up perspective view of the distal region of the bone staple delivery device of FIG. 1;
FIG. 5 is a perspective view of the medical device of FIG. 1, with the bone staple installed in the bone staple delivery device and the staple undeformed;
FIG. 6 is a top view of the medical device of FIG. 1, with the bone staple installed in the bone staple delivery device and the staple unclamped and undeformed;
FIG. 7 is a top view of the medical device of FIG. 1, with the bone staple installed in the bone staple delivery device and the staple clamped and deformed;
FIG. 8 is a closed-up perspective view of a distal region of a platform of the bone stable delivery device of FIG. 1;
FIG.9 is a perspective view of a lever-head of the bone stable delivery device of FIG. 1;
FIG.10 is another perspective of a lever-head of the bone stable delivery device of FIG. 1;
FIG. 11 is a perspective view of the lever-head and the distal region of the platform of the bone stable delivery device of FIG. 1;
FIG. 12 is a side view of a distal region of a lever-handle of the bone stable delivery device of FIG. 1;
FIG. 13 is a perspective view of a distal region of the lever-handle and the lever-head of the bone stable delivery device of FIG. 1;
FIG. 14 is another perspective view of the distal region of the lever-handle and the lever-head of the bone stable delivery device of FIG. 1;
FIG. 15 is a side view of the distal region of the lever-handle and the lever-head of the bone stable delivery device of FIG. 1;
FIG. 16 is a perspective view of another bone staple delivery device according to the present disclosure;
FIG. 17 is another perspective view of the bone staple delivery device of FIG. 16;
FIG. 18 is another perspective view of the bone staple delivery device of FIG. 16;
FIG. 19 is a perspective view of the bone staple installed in the bone staple delivery device of FIG. 16 and the staple unclamped and undeformed;
FIG. 20 is a perspective view of the bone staple installed in the bone staple delivery device of FIG. 16 and the staple clamped and deformed; and
FIG. 21 is a perspective view of another bone staple delivery device and the staple of according to the present disclosure.

### DETAILED DESCRIPTION

It may be appreciated that the present disclosure is not limited in its application to the details of construction and the arrangement of components set forth in the following description or illustrated in the drawings. The invention(s) herein may be capable of other embodiments and of being practiced or being carried out in various ways. Also, it may be appreciated that the phraseology and terminology used herein is for the purpose of description and should not be regarded as limiting as such may be understood by one of skill in the art.

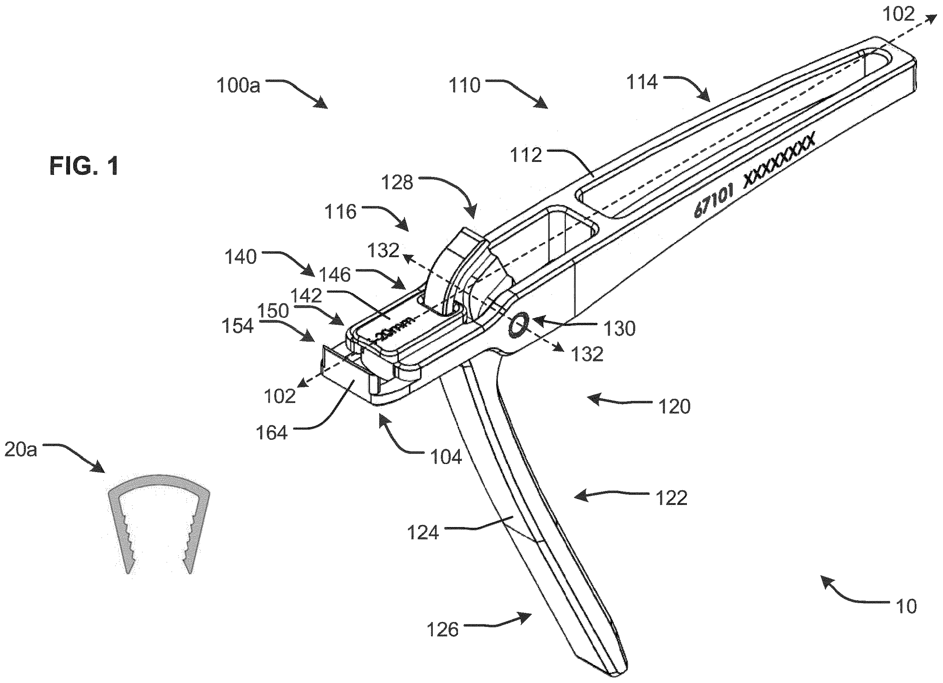

Referring now to the figures, FIG. 1 shows a medical device 10 according to the present disclosure, comprising a manually (hand-operated) bone staple delivery device 100a and a bone staple 20a. Staple delivery device 100a is used to deliver the bone staple 20a to a host, particularly to facilitate repair of a bone fracture.

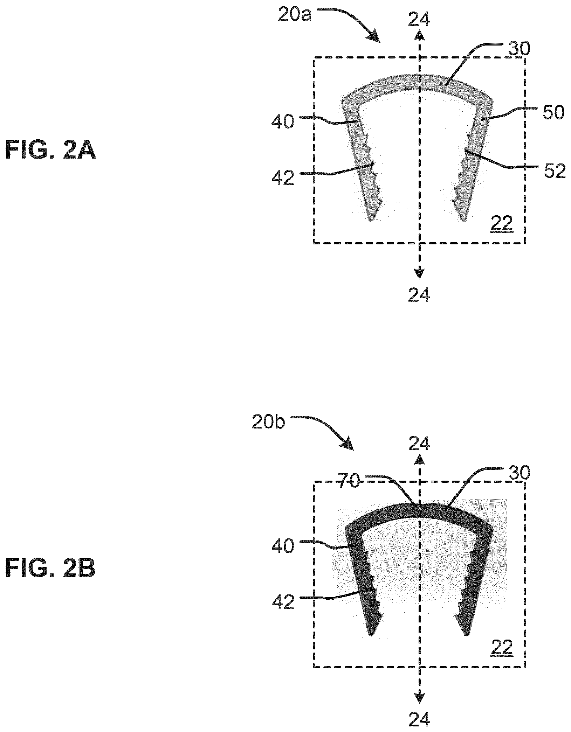

As best shown by FIG. 2A, bone staple 20a comprises an arcuate/bowed (convex) crown 30 (which may be understood to define the width of the staple) and two fastening legs 40, 50 disposed at the lateral ends of the crown 30. As shown, the crown 30 and the legs 40, 50 extend and are fully disposed in a frontal (common) plane 22, with the crown 30 being convex continually outwardly away from the legs 40, 50 as the crown 30 extends medially away from the legs 40, 50 towards a longitudinal midline 24 of the staple 20a/crown 30. Also as shown, the legs 40, 50 linearly and continually taper towards one another (converge) as the legs 40, 50 extend away from the crown 30, with the inner faces of each of the legs 40, 50 facing one another including a plurality of reverse barbs 42, 52 arranged along a longitudinal length of the legs 40, 50. Staple 20a, as well as staple 20b of FIG. 2b, may be formed of nitinol.

Returning to FIG. 1, staple delivery device 100a comprises an elongated platform 110 having a longitudinal length which extends along longitudinal axis 102 of the staple delivery device 100. As shown, elongated platform 110, which comprises an elongated platform body 112 (shown as a one-piece body), has a proximal (hand grasping end) region 114 and a distal (working end) region 116. As used herein, the terms "proximal" and "distal" may be understood as being relative to an operator (e.g. medical practitioner such as a doctor) of the staple delivery device 100a.

Staple delivery device 100a further comprises an elongated, lever-actuator 120, which both rotates/pivots relative to the platform 110, as well as moves translationally relative to the platform 110. More particularly, the lever-actuator 120 comprises a lever-handle 122 which rotates/pivots relative to the platform 110, and a lever-head 140 which moves translationally relative to the platform 110.

Lever-handle 122 has a longitudinal length which may extend along longitudinal axis 102 of the staple delivery device 100, depending on the rotational position of the lever-handle 122. Lever-handle 122 rotates about a pivot axis 132 of a pivot 130, shown provided by a pivot pin. Lever-handle 122, which comprises an elongated lever-handle body 124 (shown as a one-piece body), has a lever-handle proximal (hand grasping end) region 126 and a lever-handle distal (working end) region 128.

Translationally moveable lever-head 140 has a longitudinal length which extends along longitudinal axis 102 of the staple delivery device 100. Lever head 140 slides with linear movement along a longitudinal axis 102 of the staple delivery device 100a, which is also the longitudinal axis of the platform 110 and the lever-head 140. As shown, the longitudinal axis 102 and the pivot axis 132 are transverse, particularly perpendicular, to each other. Lever-head 140, which comprises a lever-head body 142 (shown as a one-piece body), also has a lever-head proximal region 146 and a lever-head distal region 150.

As shown, the lever-head 140, particularly the proximal region 146, is positively mechanically coupled (against separation) to the lever-handle 122, particularly the distal region 128. As explained in greater detail below, when the lever-handle 122 is rotated towards the elongated platform 110, and more particularly the proximal region 126 rotated towards the proximal region 114 of the platform 110, the lever-head 140 moves linearly distally along the longitudinal axis 102 of the staple delivery device 100a. Conversely, when the when the lever-handle 120 is rotated away from the elongated platform 110, and more particularly the proximal region 126 rotated away from the proximal region 114 of the platform 110, lever-head 140 moves linearly proximally along the longitudinal axis 102 of the staple delivery device 100a.

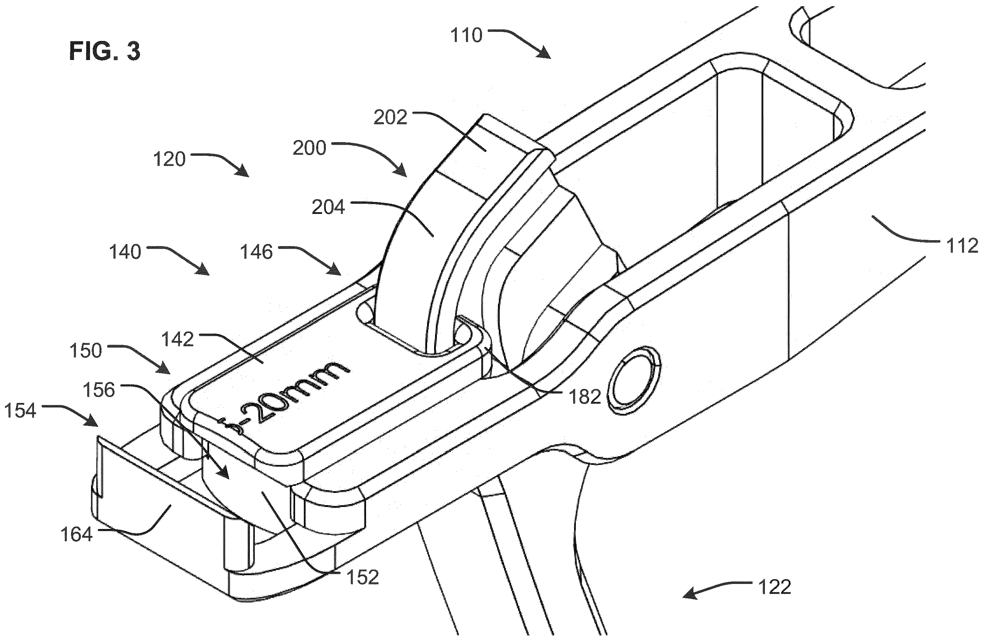

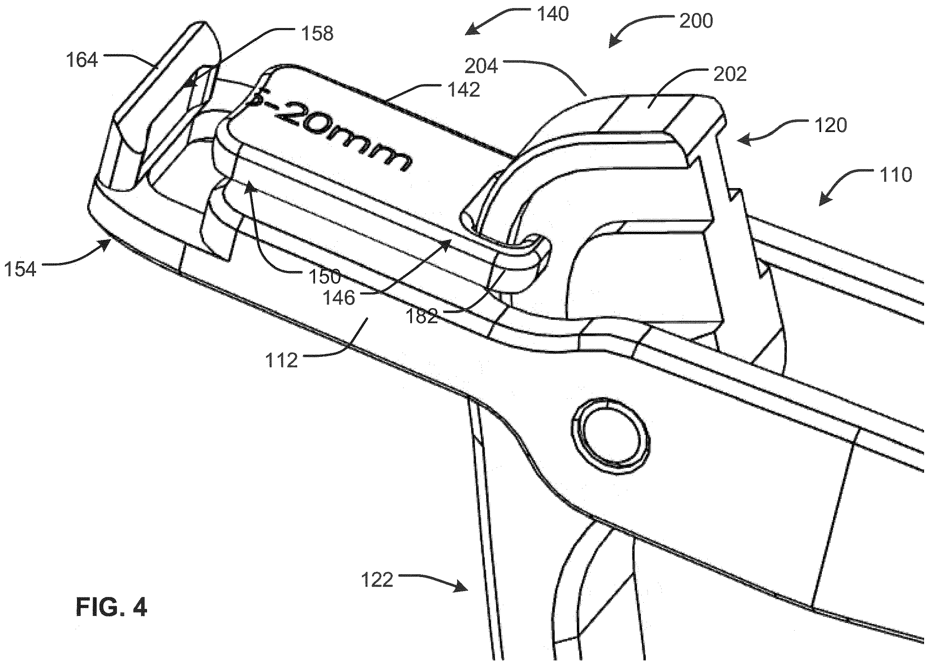

As shown in FIG. 1, the staple delivery device 100a includes staple clamp 154 at a working end 104 of the staple delivery device 100a. As better shown in FIG. 3, the staple clamp 154 comprises a opposing proximal clamp face 156 (formed by lever-head distal (end) face 152 of lever-head 140) and, as better shown in FIG. 4, distal clamp face 158 (formed by end wall 164 of the distal region 116 of platform 110).

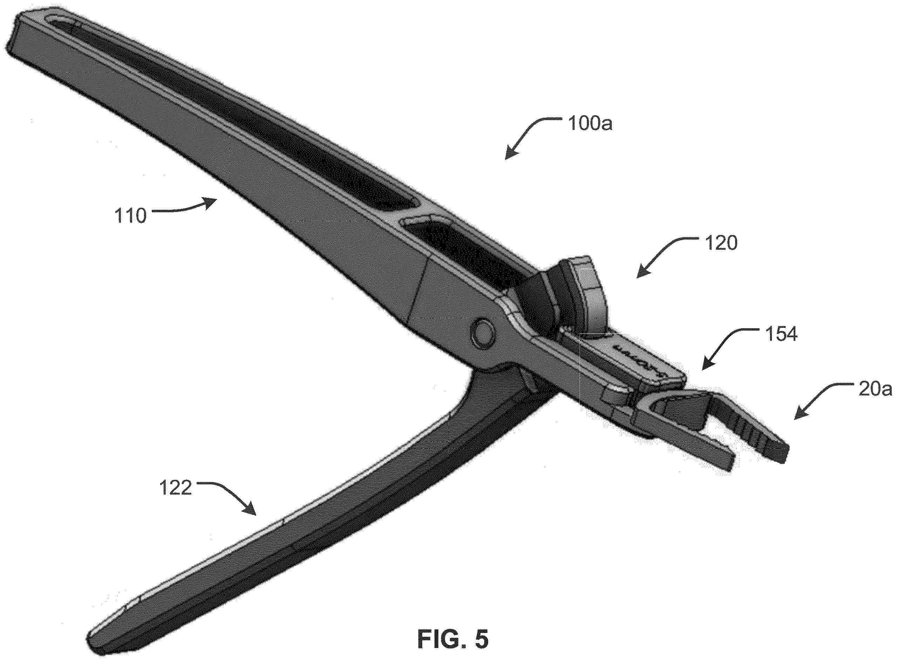

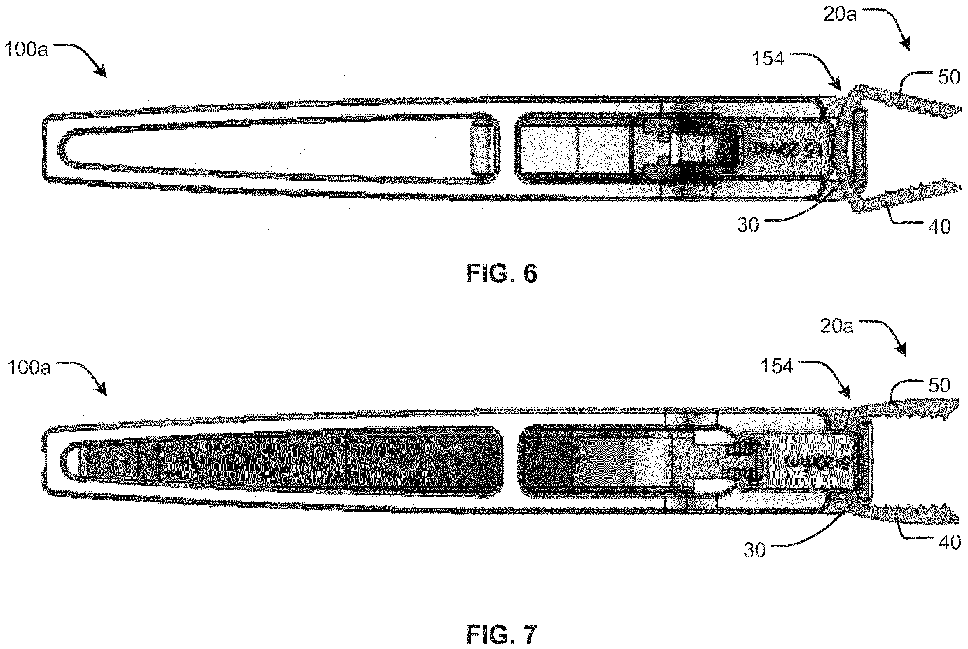

Referring to FIG. 5, there is shown staple 20a installed in clamp 154, with clamp 154 in the staple installed position (which may also be referred to as clamp open position), in which the staple 20a is in an unclamped (or undeformed) state. Referring to FIG. 6, such is a top view of FIG. 5. As shown in FIGS. 5 and 6, the legs 40, 50 linearly and continually taper towards one another as the legs 40, 50 extend away from the crown 30.

Now, referring to FIG. 7, clamp 154 in in a staple installed and clamped position (which may be referred to as the clamp closed position), in which the staple 20a is in a clamped (or elastically deformed) state. As shown, the crown 30 of the staple 20a in elastically deformed to flatten the arc/curvature of the crown 30, which, in turn, causes the legs 40, 50 of the staple 20a to splay (spread in the frontal plane 22 away from the midline 24) further apart and separate (diverge) as to become more parallel, particularly substantially parallel (e.g. within 10 degrees of being parallel). With the staple 20a arranged in this position, the staple 20a may be then disposed in a bone, with the crown 30 spanning the fracture and each of the legs 40, 50 residing in one of the adjacent bone segments. When the staple 20a is released from the staple delivery device 100a, the elastic memory of the staple 20a, and more particularly the crown 30, being urged towards its initial "bowed" state, causes the legs 40, 50 to attempt to elastically recover towards one another. The force associated with the staple legs 40, 50 being urged back towards one another due to elastic memory/recovery provides a compression force which urges the adjacent bone segments on each side of the fracture towards one another.

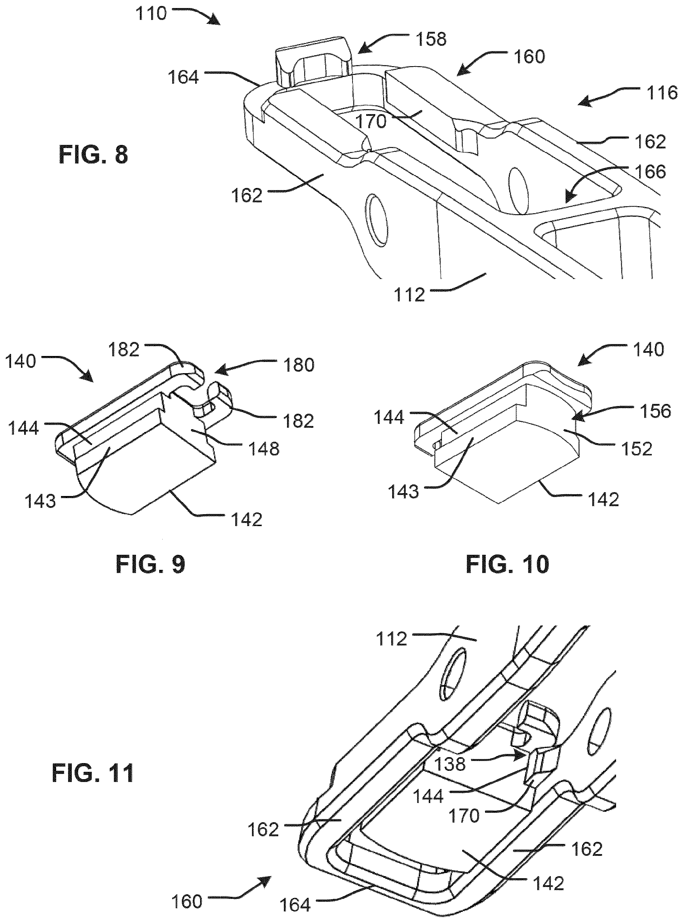

Referring now to FIGS. 8-11, the distal region 116 of the platform 110 is shown in FIG. 8, the lever-head 140 is shown in FIGS. 9-10 and the lever-head 140 assembled with the platform is shown in FIG. 11.

As shown by FIG. 8, the distal region 116 of the platform 110 comprises a U-shaped structure 160 having two longitudinally extending parallel arms 162 and a transverse distal end wall 164, which define a receiving space 166. As shown, arms 162 are mirror images of one another. The inner face of each arm 162 of the platform 110 includes a longitudinally extending protruding elongated linear rail 170, which is received in an elongated linear rail groove 144 of the lever-head 140, respectively formed on opposite lateral faces 143 of the lever-head 140. Such is shown in FIG. 11. In this manner, the linear rail 170 (which may be understood as a first connector) and the linear groove 144 (which may be understood a second mating connector which mates with the first connector) are positively mechanically coupled (against separation), particularly slidably engaged with a sliding linkage, relative to one another, and form a linear guide track 138 for proximal/distal movement of the lever-head 140.

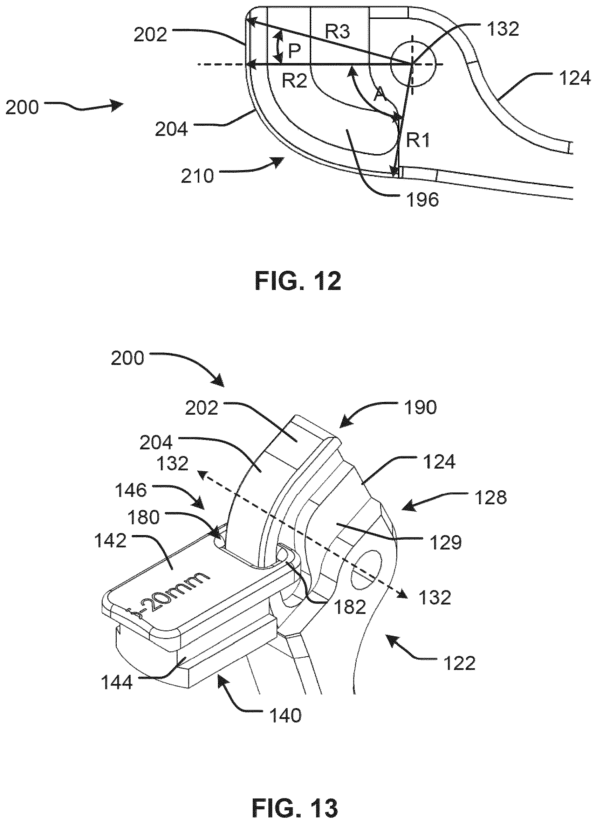

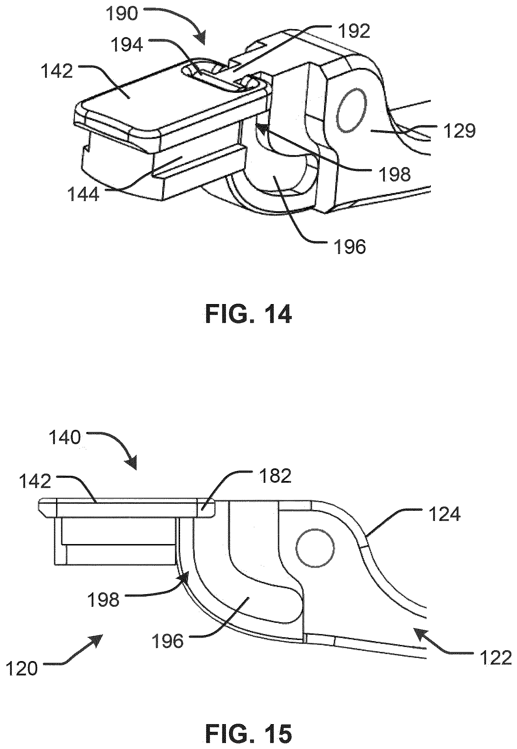

Referring now to FIGS. 12-15, the proximal region 146 of the lever-head 140 and the distal region 128 of the lever-handle 122 are mechanically coupled such that rotational motion of the lever-handle 122 about pivot axis 132 in a first direction (e.g. away from proximal end region 114 of platform 110) causes linear (translational) motion of the lever-head 140 in a first direction (e.g. proximally), and rotational motion of the lever-handle 122 about pivot axis 132 in a second direction opposite the first direction (e.g. towards proximal end region 114 of platform 110) causes linear (translational) motion of the lever-head 140 in a second direction opposite the first direction (e.g. distally).

As shown, the proximal region 146 of the lever-head 140 comprises a T-shaped slot 180, which is formed by the proximal (end) face 148 of the lever-head 140 and two opposing, laterally spaced, curved, proximal end projections 182, which are more particularly shown as limbs having an elbow-shape, particularly L-shaped, with a 90° bend. As shown, projections 182 are mirror images of one another and, as the projections 182 extend proximally, the projections 182 curve towards one another and towards the longitudinal axis 102. As shown, the T-shaped slot 180 provides a receptacle to receive a T-shaped section 190 of the lever-handle 122. As shown, the T-shaped section 190 comprises a stem 192, which extends parallel with the longitudinal axis 102, and a crossbar 194, which extends transverse (perpendicular) to the stem 192 and the longitudinal axis 102. As shown, the opposite lateral faces 129 of the distal region 128 of the lever-handle 122 each include an arcuate groove 196, the bottom of which is defined by the stem 192. As shown, each curved projection 182 of the lever-head 140 is received in a one of the arcuate grooves 196 of the lever-handle 122, respectively. In this manner, the T-shaped section 190 of the lever-handle 122 (which may be understood as a first connector) and the T-shaped slot 180 of the lever-head 140 (which may be understood a second mating connector which mates with the first connector) are positively mechanically coupled (against separation), particularly slidably engaged with a sliding linkage, relative to one another. Further, an arcuate guide track 198 is formed by the projections 182 of the lever-head 140 being disposed in the grooves 196 of the lever-handle 122.

During operation, the proximal end face 148 of the lever-head 140 and the distal end face 200 of the lever-handle 122, operatively interact (contact) with one another. As shown, the proximal end face 148 of the lever-head 140 is planar (flat). As further shown, the distal end face 200 of the lever-handle 122 comprises a planar end face 202 and an arcuate end face 204 adjacent one another, which both extend a distance about (around) the pivot axis 132

Arcuate end face 204 is a cam surface, of a cam 210 which has a continually increasing radial distance to the pivot axis 132 from radial distance R1 to radial distance R2. As shown, the increasing radial distance extends over subtended angle A of the arcuate end face 204, having the pivot axis 132 as the vertex. Stated another way, angle A is the angle subtended by the arcuate end face 204 from the vertex/pivot axis 132. As shown, the arcuate end face subtended angle A is an acute angle in a range of 75-85 degrees about the pivot axis 132. With regards to the planar end face 202, such extends from radial distance R2 to radial distance R3 over subtended angle P of the planar end face 202, also having the pivot axis 132 as the vertex. Stated another way, angle P is the angle subtended by the planar end face 202 from the vertex/pivot axis 132. As shown, the planar end face subtended angle P is an acute angle in a range of 20-30 degrees about the pivot axis 132.

As a result of the increasing radial distance over angle A as the proximal region 126 o the lever-handle 122 is rotated with a closure force towards the proximal region 114 of the platform 110, the arcuate end face 204 of the working region 128 of the lever-handle 122 contacts with and pushes against the planar proximal end face 148 of the lever-head 140, which force moves the lever-head 140 linearly distally along the longitudinal axis 102 of the staple delivery device 100a. During the rotation, the lever head 140 moves along the linear guide track 138 formed by the lever-head 140 and the platform 110, and also moves along the arcuate guide track 198 formed by the lever-head 140 and the lever-handle 122.

As the lever-head 140 moves distally, the clamp 154, having proximal clamp face 156 formed by lever-head distal face 152 of lever-head 140) and distal clamp face 158 formed by end wall 164 of the distal region 116 of platform 110), closes and clamps crown 30 of staple 20a. As the clamp 154 closes and clamps crown 30 of staple 20a, the crown 30 of the staple 20a in elastically deformed to flatten the arc/curvature of the crown 30, which, in turn, causes the legs 40, 50 of the staple 20a to splay (spread in the frontal plane 22 away from the midline 24) further apart and separate (diverge) as to become more parallel.

It should be noted that, as the lever-handle 122 is rotated towards the elongated platform 110, and the arcuate end face 204 of the working region 128 of the lever handle 122 pushes against the planar proximal end face 148 of the lever-head 140, a bias force is applied to the lever-handle 122 by the elastically deformed staple 20a which urges the lever-handle 122 to rotate in the opposite direction. Thus, if the manual (hand-applied) force to clamp the staple 20a is removed while the arcuate end face 204 of the working region 128 of the lever handle 122 is acting on the planar proximal end face 148 of the lever-head 140 (e.g. the staple delivery device 100a is no longer held by the operator), the lever-handle 122 will rotate away from the platform 110 and the clamping force on the staple 20a will be lost.

However, as the lever-handle 122 is further rotated towards the elongated platform 110, the planar end face 202 of the working region 128 of the lever handle 122 aligns with the planar proximal end face 148 of the lever-head 140 subsequent to the arcuate end face 204, such that the end faces 148, 202 face one another and become parallel, with the resilient (bias) force of the staple 20a forcing the planar end face 148 of the lever-head 140 towards and into contact with the planar end face 202 of the lever-handle 122.

In the foregoing manner, once the arcuate end face 204 of the lever-handle 140 is no longer in contact with the planar proximal end face 148 of the lever-head 140, the lever-actuator 120 is now held and maintained stationary against the resilient (bias) force of the staple 20a acting on the level actuator 120. Thus, in this state, even if the staple delivery device 100a is no longer held by the operator, the staple 20a will remain clamped with the legs 40, 50 splayed for insertion into a bone.

When the legs 40, 50 are splayed and inserted into a bone, the lever-handle 22 may then be rotated in the opposite direction, i.e. away from the elongated platform 110, to open the clamp 154 and release the staple 20a. As the lever-handle 122 is rotate away from the elongated platform 110, the projections 182 of the lever-head 140 move within the grooves 196 of the lever-handle 122, and the T-shaped section 190 of the lever-handle 122 pulls the lever-head 140 proximally.

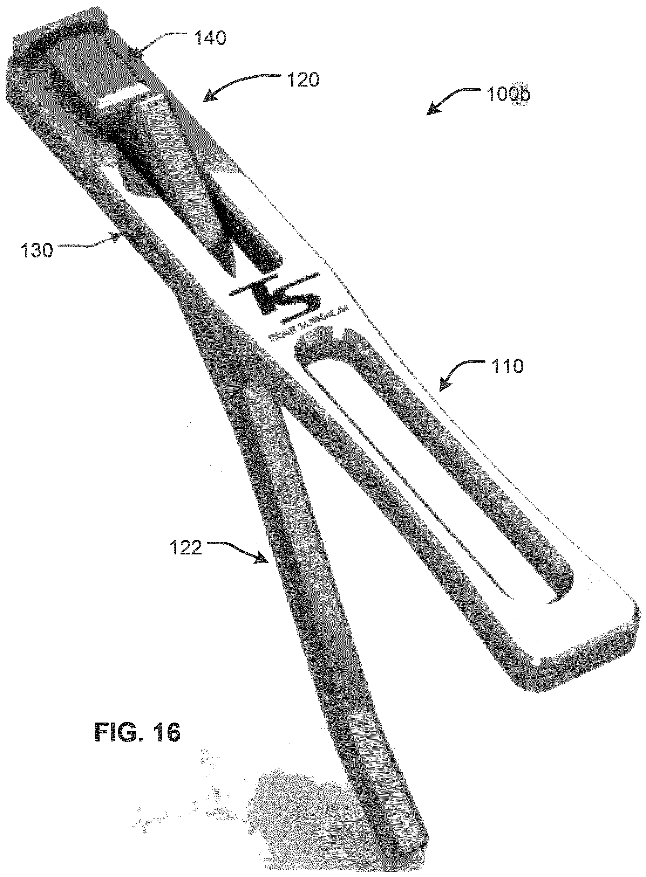

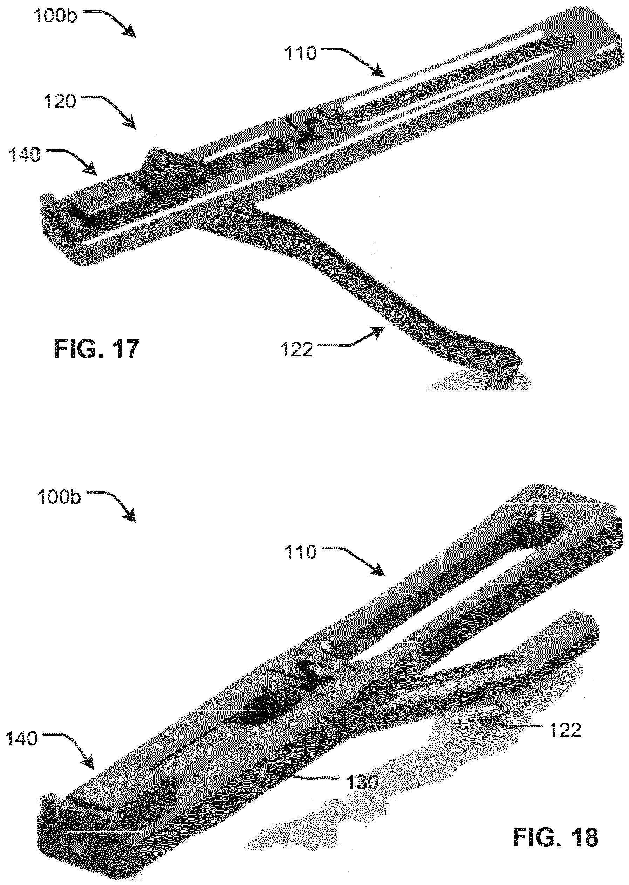

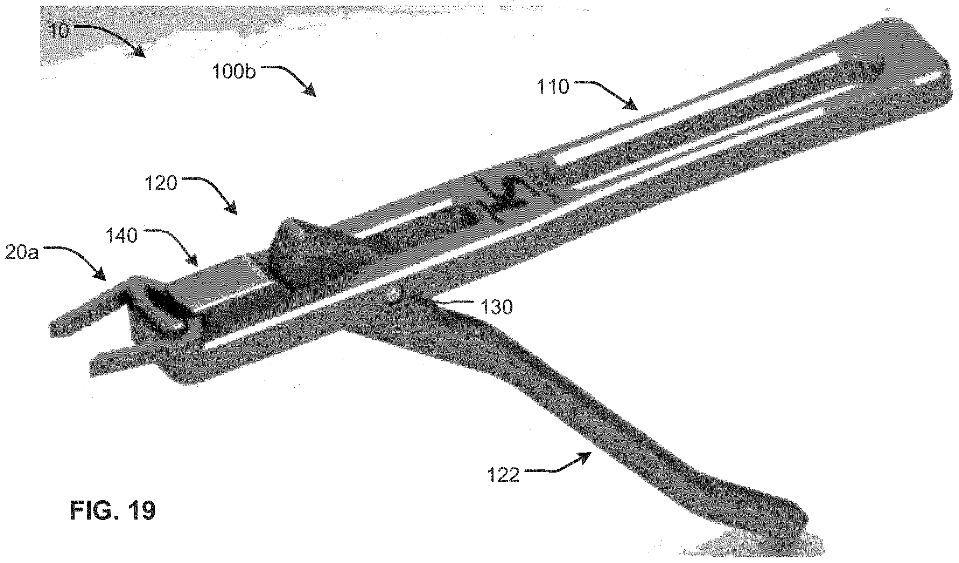

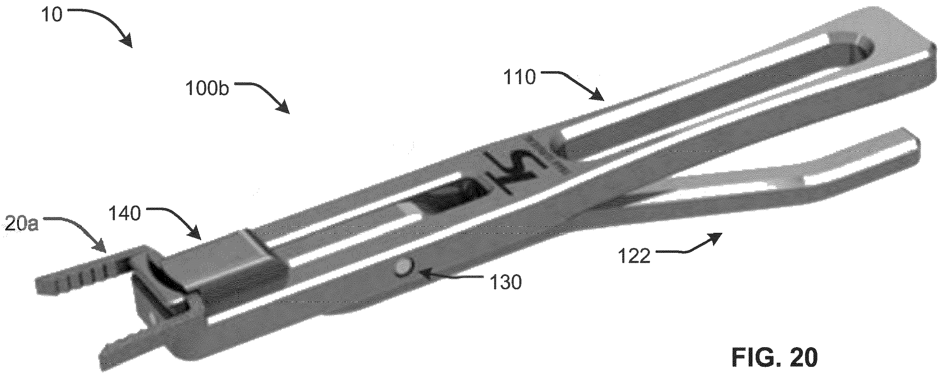

Turning to the embodiment of FIGS. 16-20, with staple delivery device 100b, the lever-head 140 is no longer mechanically coupled to the lever-handle 122. Similar to the prior embodiment, when the proximal region 126 of the lever-handle 122 rotated towards the proximal region 114 of the platform 110, the lever-head 140 moves linearly distally along the longitudinal axis 102 of the staple delivery device 100b. However, when the proximal region 126 of the lever-handle 122 rotated away from the proximal region 114 of the platform 110, lever-head 140 will not move linearly proximally along the longitudinal axis 102 of the staple delivery device 100b due to the absence of the mechanical coupling of the lever-head 140 and the lever-handle 112. Rather, lever-head 140 must be moved manually (by hand).

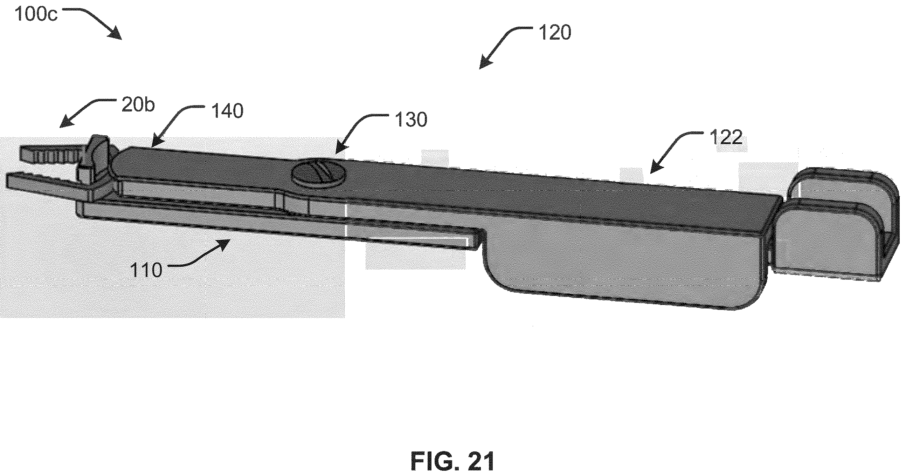

Turning to the embodiment of FIG. 21, such makes use of staple 20b of FIG. 2B, which includes a crown detent 70 to receive the lever-head 140 which is now formed as one-piece with the lever-handle 122. Also as shown, the lever-handle 122 rotates parallel to the platform 110, about pivot screw 130. Thus, the embodiment of FIG. 21 operates in a "scissor-like" manner to rotate the lever handle 122 relative to the platform 110.

While a preferred embodiment of the present invention(s) has been described, it should be understood that various changes, adaptations and modifications can be made therein without departing from the scope of the appended claims. The scope of the invention(s) should, therefore, be determined not with reference to the above description, but instead should be determined with reference to the appended claims .

### Listing of reference characters:

- 10: medical device
- 20: bone staple (20a. 20b)
- 22: plane
- 24: midline
- 30: crown
- 40: leg
- 42: barb
- 50: leg
- 52: barb
- 70: detent
- 100: bone staple delivery device (100a, 100b, 100c)
- 102: longitudinal axis
- 104: working end
- 110: platform
- 112: platform body
- 114: platform proximal (grasping end) region
- 116: platform distal (working end) region
- 120: lever-actuator
- 122: lever-handle
- 124: lever-handle body
- 126: lever-handle proximal (grasping end) region
- 128: lever-handle distal (working end) region
- 129: lever-handle lateral face
- 130: pivot
- 132: pivot axis
- 138: guide track
- 140: lever-head
- 142: lever-head body
- 143: lever-head lateral face
- 144: lever-head groove
- 146: lever-head proximal region
- 148: lever-head proximal (end) face
- 150: lever-head distal (end) region
- 152: lever-head distal (end) face
- 154: clamp
- 156: proximal clamp face
- 158: distal clamp face
- 160: platform body U-shaped structure
- 162: platform arms
- 164: platform transverse end wall
- 166: platform receiving space
- 170: platform rail
- 180: lever-head T-shaped slot
- 182: lever-head projections
- 190: lever-handle T-shaped section
- 192: stem
- 194: crossbar
- 196: groove
- 198: arcuate guide track
- 200: lever-handle distal end face
- 202: planar end face
- 204: arcuate end face
- 210: cam
- A: arcuate end face subtended angle
- P: planar end face subtended angle
- R1: radial distance 1
- R2: radial distance 2
- R3: radial distance 3.

## Claims

1. A medical device (10), comprising:
a bone staple delivery device (100a), the bone staple delivery device (100a) comprising an elongated platform (110) and a lever-actuator (120);
the platform (110) and the lever-actuator (120) forming a staple clamp (154) to clamp a bone staple (20a);
the lever-actuator (120) having a lever-handle (122) and a lever-head (140), the lever-handle (122) rotatable relative to the platform (110) and having a distal end face (200) comprising a planar distal end face (202) and an arcuate distal end face (204);
the lever-head (140) having a planar proximal end face (148);
**characterized in that**, when the staple (20a) is installed in the staple clamp (154) and the lever-handle (122) is rotated with a closure force in a first direction towards the platform (110), the bone staple delivery device (100a) is operable such that the arcuate distal end face (204) of the lever-handle (122) contacts the planar proximal end face (148) of the lever-head (140) and moves the lever-head (140) distally relative to the platform (110) to close the staple clamp (154) on the staple (20a); and
**in that**, when the lever-handle (122) is further rotated with the closure force in the first direction towards the platform (110), the planar distal end face (202) of the lever-handle (122) and the planar proximal end face (148) of the lever-head (140) come into parallel alignment facing one another, which inhibits the lever-handle (122) from rotating in a second direction away from the platform (110) upon removal of the closure force.

2. The medical device of claim 1, wherein:
the lever-handle (122) is rotatable relative to the platform (110) about a pivot axis (132): and
the arcuate distal end face (204) of the lever-handle (122) extends around the pivot axis (132) with an increasing radial distance from the pivot axis (132).

3. The medical device of claim 1, wherein:
the planar distal end face (202) of the lever-handle (122) is adjacent the arcuate distal end face (204) of the lever-handle (122).

4. The medical device of claim 1, wherein:
an angle subtended by the arcuate distal end face (204) of the lever-handle (122) from the pivot axis (132) is in a range of 75-85 degrees, and/or
an angle subtended by the planar distal end face (202) of the lever-handle (122) from the pivot axis (132) is in a range of 20-30 degrees.

5. The medical device of claim 1, wherein:
the bone staple delivery device (100a) has a longitudinal axis (102):
the platform (110) extends along the longitudinal axis (102), and comprises a platform distal region (116); and
the platform distal region (116) comprises two longitudinally extending arms (162) spaced apart by a receiving space which contains the lever-head (140).

6. The medical device of claim 5, wherein:
the lever-head (140) and the platform distal region (116) form at least one linear guide track (138) along which the lever-head (140) is movable proximally and distally along the longitudinal axis (102) of the bone staple delivery device (100a).

7. The medical device of claim 6, wherein:
the at least one linear guide track (138) comprises a linear rail (170) disposed in a linear groove (144), and optionally wherein the platform distal end forms the linear rail (170) and the lever-head (140) forms the linear groove (144).

8. The medical device of claim 6, wherein:
the at least one linear guide track (138) comprises two linear guide tracks (138) along which the lever-head (140) is movable proximally and distally along the longitudinal axis (102) of the bone staple delivery device (100a), and optionally wherein each of the two linear guide tracks (138) is formed by one of the longitudinally extending arms (162) of the platform distal region (116) and a lateral face of the lever-head (140), respectively.

9. The medical device of claim 5, wherein:
the lever-handle (122) comprises a lever-handle distal region (128);
the receiving space contains the lever-handle distal region (128);
the lever-head (140) and the lever-handle distal region (128) form at least one arcuate guide track (198) along which the lever-handle distal region (128) is rotatable relative to the lever-head (140).

10. The medical device of claim 9, wherein:
the at least one arcuate guide track (198) comprises a projection (182) disposed in an arcuate groove (196), and optionally wherein the lever-head (140) forms the projection (182) and the lever-handle distal region (128) forms the arcuate groove (196).

11. The medical device of claim 9, wherein:
the at least one arcuate guide track (198) comprises two arcuate guide tracks (198) along which the lever-handle distal region (128) is rotatable relative to the lever-head (140), and optionally wherein each of the two arcuate guide tracks (198) is formed by a proj ection of the lever-head (140) and a lateral face of the lever-handle distal region (128), respectively.

12. The medical device of claim 8, wherein:
the lever-handle (122) and the lever-head (140) are mechanically coupled by a T-shaped section of the lever-handle (122) disposed on a T-shaped slot of the lever-head (140).

13. The medical device of claim 1, wherein:
the platform (110) comprises a platform proximal region (114);
the lever-handle (122) comprises a lever-handle proximal region (126); and
each of the platform proximal region (114) and the lever-handle proximal region (126) comprise a hand-grasping region, respectively.

14. The medical device of claim 1, wherein:
the bone staple delivery device (100a) is manually operable.

15. The medical device of claim 1, further comprising:
the bone staple (20a) installable in the bone staple delivery device (100a).

## Patentansprüche

1. Medizinische Vorrichtung (10), umfassend:
eine Knochenklammer-Setzvorrichtung (100a), wobei die Knochenklammer-Setzvorrichtung (100a) eine langgestreckte Plattform (110) und eine Hebelbetätigungseinrichtung (120) umfasst;
wobei die Plattform (110) und die
Hebelbetätigungseinrichtung (120) eine Klammerklemme (154) zum Einklemmen einer Knochenklammer (20a) bilden; wobei die Hebelbetätigungseinrichtung (120) einen Hebelgriff (122) und einen Hebelkopf (140) aufweist, wobei der Hebelgriff (122) relativ zu der Plattform (110) drehbar ist und eine distale Stirnfläche (200) aufweist, die eine ebene distale Stirnfläche (202) und eine bogenförmige distale Stirnfläche (204) umfasst; wobei der Hebelkopf (140) eine ebene proximale Stirnfläche (148) aufweist;
**dadurch gekennzeichnet,**
**dass**, wenn die Klammer (20a) in die Klammerklemme (154) eingesetzt ist und der Hebelgriff (122) unter Einwirkung einer Schließkraft in einer ersten Richtung auf die Plattform (110) zu gedreht wird, die Knochenklammer-Setzvorrichtung (100a) derart betätigbar ist, dass die bogenförmige distale Stirnfläche (204) des Hebelgriffs (122) die ebene proximale Stirnfläche (148) des Hebelkopfs (140) berührt und den Hebelkopf (140) relativ zu der Plattform (110) in distaler Richtung bewegt, um die Klammerklemme (154) um die Klammer (20a) zu schließen; und
**dass**, wenn der Hebelgriff (122) unter Einwirkung der Schließkraft in der ersten Richtung weiter auf die Plattform (110) zu gedreht wird, die ebene distale Stirnfläche (202) des Hebelgriffs (122) und die ebene proximale Stirnfläche (148) des Hebelkopfs (140) in eine einander zugewandte parallele Ausrichtung gelangen, was verhindert, dass der Hebelgriff (122) bei Wegnahme der Schließkraft in einer zweiten Richtung von der Plattform (110) weg gedreht wird.

2. Medizinische Vorrichtung nach Anspruch 1, wobei:
der Hebelgriff (122) relativ zu der Plattform (110) um eine Schwenkachse (132) drehbar ist; und
sich die bogenförmige distale Stirnfläche (204) des Hebelgriffs (122) um die Schwenkachse (132) herum erstreckt, wobei der radiale Abstand von der Schwenkachse (132) zunimmt.

3. Medizinische Vorrichtung nach Anspruch 1,
bei welcher die ebene distale Stirnfläche (202) des Hebelgriffs (122) der bogenförmigen distalen Stirnfläche (204) des Hebelgriffs (122) benachbart ist.

4. Medizinische Vorrichtung nach Anspruch 1,
bei welcher ein durch die bogenförmige distale Stirnfläche (204) des Hebelgriffs (122) von der Schwenkachse (132) aus aufgespannter Winkel in einem Bereich von 75 bis 85 Grad liegt und/oder
ein durch die ebene distale Stirnfläche (202) des Hebelgriffs (122) von der Schwenkachse (132) aus aufgespannter Winkel in einem Bereich von 20 bis 30 Grad liegt.

5. Medizinische Vorrichtung nach Anspruch 1,
bei welcher die Knochenklammer-Setzvorrichtung (100a) eine Längsachse (102) aufweist;
sich die Plattform (110) entlang der Längsachse (102) erstreckt und einen distalen Plattformbereich (116) umfasst; und
der distale Plattformbereich (116) zwei sich in Längsrichtung erstreckende Arme (162) umfasst, die durch einen Aufnahmeraum voneinander beabstandet sind, welcher den Hebelkopf (140) enthält.

6. Medizinische Vorrichtung nach Anspruch 5,
bei welcher der Hebelkopf (140) und der distale Plattformbereich (116) wenigstens eine lineare Führungsbahn (138) bilden, entlang derer der Hebelkopf (140) entlang der Längsachse (102) der Knochenklammer-Setzvorrichtung (100a) proximal und distal bewegbar ist.

7. Medizinische Vorrichtung nach Anspruch 6,
bei welcher die wenigstens eine lineare Führungsbahn (138) eine in einer linearen Nut (144) angeordnete lineare Schiene (170) umfasst, und wobei optional das distale Ende der Plattform die lineare Schiene (170) bildet und der Hebelkopf (140) die lineare Nut (144) ausbildet.

8. Medizinische Vorrichtung nach Anspruch 6,
bei welcher die wenigstens eine lineare Führungsbahn (138) zwei lineare Führungsbahnen (138) umfasst, entlang derer der Hebelkopf (140) entlang der Längsachse (102) der Knochenklammer-Setzvorrichtung (100a) proximal und distal bewegbar ist, und wobei optional jede der beiden linearen Führungsbahnen (138) jeweils durch einen der sich in Längsrichtung erstreckenden Arme (162) des distalen Plattformbereichs (116) und eine Seitenfläche des Hebelkopfs (140) gebildet ist.

9. Medizinische Vorrichtung nach Anspruch 5,
bei welcher der Hebelgriff (122) einen distalen Hebelgriffbereich (128) umfasst;
der Aufnahmeraum den distalen Hebelgriffbereich (128) enthält;
der Hebelkopf (140) und der distale Hebelgriffbereich (128) wenigstens eine bogenförmige Führungsbahn (198) ausbilden, entlang derer der distale Hebelgriffbereich (128) relativ zu dem Hebelkopf (140) drehbar ist.

10. Medizinische Vorrichtung nach Anspruch 9,
bei welcher die wenigstens eine bogenförmige Führungsbahn (198) einen in einer bogenförmigen Nut (196) angeordneten Vorsprung (182) umfasst, und wobei optional der Hebelkopf (140) den Vorsprung (182) bildet und der distale Hebelgriffbereich (128) die bogenförmige Nut (196) ausbildet.

11. Medizinische Vorrichtung nach Anspruch 9,
bei welcher die wenigstens eine bogenförmige Führungsbahn (198) zwei bogenförmige Führungsbahnen (198) umfasst, entlang derer der distale Hebelgriffbereich (128) relativ zu dem Hebelkopf (140) drehbar ist, und wobei optional jede der beiden bogenförmigen Führungsbahnen (198) jeweils durch einen Vorsprung des Hebelkopfs (140) und eine Seitenfläche des distalen Hebelgriffbereichs (128) ausgebildet ist.

12. Medizinische Vorrichtung nach Anspruch 8,
bei welcher der Hebelgriff (122) und der Hebelkopf (140) durch einen T-förmigen Abschnitt des Hebelgriffs (122), der an einem T-förmigen Schlitz des Hebelkopfs (140) angeordnet ist, mechanisch gekoppelt sind.

13. Medizinische Vorrichtung nach Anspruch 1,
bei welcher die Plattform (110) einen proximalen Plattformbereich (114) umfasst;
der Hebelgriff (122) einen proximalen Hebelgriffbereich (126) umfasst; und
der proximale Plattformbereich (114) und der proximale Hebelgriffbereich (126) jeweils einen Handgreifbereich umfassen.

14. Medizinische Vorrichtung nach Anspruch 1,
bei welcher die Knochenklammer-Setzvorrichtung (100a) manuell betätigbar ist.

15. Medizinische Vorrichtung nach Anspruch 1,
welche weiter die Knochenklammer (20a) umfasst, die in die Knochenklammer-Setzvorrichtung (100a) einsetzbar ist.

## Revendications

1. Dispositif médical (10), comprenant:
un dispositif de pose d'agrafe osseuse (100a), le dispositif de pose d'agrafe osseuse (100a) comprenant une plateforme allongée (110) et un actionneur à levier (120);
la plateforme (110) et l'actionneur à levier (120) formant une pince à agrafe (154) pour pincer une agrafe osseuse (20a);
l'actionneur à levier (120) ayant une poignée de levier (122) et une tête de levier (140), la poignée de levier (122) pouvant être amenée à tourner par rapport à la plateforme (110) et ayant une face d'extrémité distale (200) comprenant une face d'extrémité distale plane (202) et une face d'extrémité distale arquée (204);
la tête de levier (140) ayant une face d'extrémité proximale plane (148);
**caractérisé en ce que**,
lorsque l'agrafe (20a) est installée dans la pince à agrafe (154) et que la poignée de levier (122) est amenée à tourner avec une force de fermeture dans un premier sens vers la plateforme (110), le dispositif de pose d'agrafe osseuse (100a) peut être actionné de telle sorte que la face d'extrémité distale arquée (204) de la poignée de levier (122) entre en contact avec la face d'extrémité proximale plane (148) de la tête de levier (140) et déplace la tête de levier (140) de manière distale par rapport à la plateforme (110) pour fermer la pince à agrafe (154) sur l'agrafe (20a); et
**en ce que**,
lorsque la poignée de levier (122) est amenée à tourner encore avec la force de fermeture dans le premier sens vers la plateforme (110), la face d'extrémité distale plane (202) de la poignée de levier (122) et la face d'extrémité proximale plane (148) de la tête de levier (140) viennent en alignement parallèle en étant tournées l'une vers l'autre, ce qui empêche la poignée de levier (122) de tourner dans un second sens en s'éloignant de la plateforme (110) lors du retrait de la force de fermeture.

2. Dispositif médical selon la revendication 1, dans lequel:
la poignée de levier (122) peut être amenée à tourner par rapport à la plateforme (110) autour d'un axe de pivotement (132); et
la face d'extrémité distale arquée (204) de la poignée de levier (122) s'étend autour de l'axe de pivotement (132) avec une distance radiale croissante depuis l'axe de pivotement (132).

3. Dispositif médical selon la revendication 1, dans lequel:
la face d'extrémité distale plane (202) de la poignée de levier (122) est adjacente à la face d'extrémité distale arquée (204) de la poignée de levier (122).

4. Dispositif médical selon la revendication 1, dans lequel:
un angle sous-tendu par la face d'extrémité distale arquée (204) de la poignée de levier (122) depuis l'axe de pivotement (132) est dans une plage de 75 à 85 degrés, et/ou
un angle sous-tendu par la face d'extrémité distale plane (202) de la poignée de levier (122) depuis l'axe de pivotement (132) est dans une plage de 20 à 30 degrés.

5. Dispositif médical selon la revendication 1, dans lequel:
le dispositif de pose d'agrafe osseuse (100a) a un axe longitudinal (102):
la plateforme (110) s'étend le long de l'axe longitudinal (102) et comprend une région distale de plateforme (116); et
la région distale de plateforme (116) comprend deux bras s'étendant longitudinalement (162) espacés par un espace de réception qui contient la tête de levier (140).

6. Dispositif médical selon la revendication 5, dans lequel:
la tête de levier (140) et la région distale de plateforme (116) forment au moins une voie de guidage linéaire (138) le long de laquelle la tête de levier (140) peut être déplacée de manière proximale et distale le long de l'axe longitudinal (102) du dispositif de pose d'agrafe osseuse (100a).

7. Dispositif médical selon la revendication 6, dans lequel:
la au moins une voie de guidage linéaire (138) comprend un rail linéaire (170) disposé dans une rainure linéaire (144), et éventuellement dans lequel l'extrémité distale de plateforme forme le rail linéaire (170) et la tête de levier (140) forme la rainure linéaire (144).

8. Dispositif médical selon la revendication 6, dans lequel:
la au moins une voie de guidage linéaire (138) comprend deux voies de guidage linéaires (138) le long desquelles la tête de levier (140) peut être déplacée de manière proximale et distale le long de l'axe longitudinal (102) du dispositif de pose d'agrafe osseuse (100a), et éventuellement dans lequel chacune des deux voies de guidage linéaires (138) est formée par l'un des bras s'étendant longitudinalement (162) de la région distale de plateforme (116) et par une face latérale de la tête de levier (140), respectivement.

9. Dispositif médical selon la revendication 5, dans lequel:
la poignée de levier (122) comprend une région distale de poignée de levier (128);
l'espace de réception contient la région distale de poignée de levier (128);
la tête de levier (140) et la région distale de poignée de levier (128) forment au moins une voie de guidage arquée (198) le long de laquelle la région distale de poignée de levier (128) peut être amenée à tourner par rapport à la tête de levier (140).

10. Dispositif médical selon la revendication 9, dans lequel:
la au moins une voie de guidage arquée (198) comprend une saillie (182) disposée dans une rainure arquée (196), et éventuellement dans lequel la tête de levier (140) forme la saillie (182) et la région distale de poignée de levier (128) forme la rainure arquée (196).

11. Dispositif médical selon la revendication 9, dans lequel:
la au moins une voie de guidage arquée (198) comprend deux voies de guidage arquées (198) le long desquelles la région distale de poignée de levier (128) peut être amenée à tourner par rapport à la tête de levier (140), et éventuellement dans lequel chacune des deux voies de guidage arquées (198) est formée par une saillie de la tête de levier (140) et par une face latérale de la région distale de poignée de levier (128), respectivement.

12. Dispositif médical selon la revendication 8, dans lequel:
la poignée de levier (122) et la tête de levier (140) sont couplées mécaniquement par une section en forme de T de la poignée de levier (122) disposée sur une fente en forme de T de la tête de levier (140).

13. Dispositif médical selon la revendication 1, dans lequel:
la plateforme (110) comprend une région proximale de plateforme (114);
la poignée de levier (122) comprend une région proximale de poignée de levier (126); et
chacune de la région proximale de plateforme (114) et de la région proximale de poignée de levier (126) comprend une région de préhension manuelle, respectivement.

14. Dispositif médical selon la revendication 1, dans lequel:
le dispositif de pose d'agrafe osseuse (100a) peut être actionné manuellement.

15. Dispositif médical selon la revendication 1, comprenant en outre:
l'agrafe osseuse (20a) pouvant être installée dans le dispositif de pose d'agrafe osseuse (100a).
